# EUROPÄISCHE PATENTSCHRIFT

(11) **EP 1 152 751 B1**
(45) Veröffentlichungstag und Bekanntmachung des Hinweises auf die Patenterteilung: **20.04.2005**
(21) Anmeldenummer: 00914071.6
(22) Anmeldetag: 08.02.2000
(51) Int. Cl.: A61K 9/70, A61K 31/505, A61P 25/28

(54) **DESOXYPEGANIN-TTS**
DESOXYPEGANINE TRANSDERMAL THERAPEUTIC SYSTEM
SYSTEME THERAPEUTIQUE TRANSDERMIQUE CONTENANT DE LA DESOXYPEGANINE

(30) Priorität: 19.02.1999 DE 19906977
(43) Veröffentlichungstag der Anmeldung: 14.11.2001
(73) Patentinhaber: LTS Lohmann Therapie-Systeme AG, 56626 Andernach (DE); HF ARZNEIMITTELFORSCHUNG GmbH, 59368 Werne (DE)
(72) Erfinder: HILLE, Thomas, D-56567 Neuwied (DE); DEURER, Lothar, D-56077 Koblenz (DE); Dr. MOORMANN, Joachim, D-59368 Werne (DE)
(74) Vertreter: Schmidt, Werner, Dr.
(86) Internationale Anmeldenummer: PCT/EP2000/000971
(87) Internationale Veröffentlichungsnummer: WO 2000/048579

(56) Entgegenhaltungen:
- WO-A-94/16707
- CHEMICAL ABSTRACTS, vol. 121, no. 8, 22. August 1994 (1994-08-22) Columbus, Ohio, US; abstract no. 91450, SADIKOVA, SH. A. ET AL: "Deoxypeganine injection solution" XP002140386 & KHIM.-FARM. ZH. (1993), 27(12), 52-5 ,
- DATABASE WPI Section Ch, Week 197913 Derwent Publications Ltd., London, GB; Class B02, AN 1979-25213B XP002140387 & SU 605 614 A (AS UZB CHEM GROWING), 6. April 1978 (1978-04-06)

## Beschreibung

Die Erfindung betrifft ein transdermales therapeutisches System (TTS), das als aktiven Bestandteil Desoxypeganin (1,2,3,9-Tetrahydropyrrolo[2,1-b]chinazolin) enthält.

Aufgrund seiner pharmakologischen Eigenschaften gehört Desoxypeganin zur Gruppe der reversibel wirkenden Cholinesterasehemmstoffe. Es steht in seinen Wirkungen dem Physostigmin, dem Neostigmin und dem Galanthamin nahe, besitzt jedoch auch spezifische Eigenschaften. Desoxypeganin hemmt nicht nur die Acetylcholinesterase, sondern auch die Monoaminoxydase.

Dieser Vorteil wiegt seine dosisbezogenen etwas geringere Cholinesterasehemmwirkung auf.

Die Therapie der Alzheimerschen Krankheit erfordert langwirksame und den besonderen Umständen dieser Krankheit Rechnung tragende Arzneiformen. Schwierige Therapieschemata oder Dauerinfusionen kommen aus naheliegenden Gründen nicht in Frage. Unter einer solchen Therapie im Sinne der vorliegenden Erfindung ist eine medikamentöse Behandlung von Demenzerkrankungen (insbesondere Alzheimersche Demenz) zu verstehen, die zur Beeinflussung der geistigen Leistungsfähigkeit und / oder zur Behandlung von Begleitsymptomen angewandt wird.

Vielmehr ist ein TTS die Arzneiform der Wahl; dennoch ist es bis heute nicht gelungen, Desoxypeganin transdermal in der erforderlichen Menge zur Resorption zu bringen.

WO 94/16707 offenbart ein transdermal therapeutisches System zur Verabreichung von Galanthamin an die Haut mit einer wirkstoffundurchlässigen Rückschicht und einer haftklebenden Reservoirschicht, wobei die Reservoirschicht 40-80 Gew.-% Polymermaterial und 0,1-30 Gew.-% Galanthamin enthält. Das TTS gewährleistet eine Abgabe von Galanthamin über einen Zeitraum von mindestens 24 Stunden.

Aufgabe der Erfindung ist daher die Bereitstellung von Desoxypeganin und / oder eines seiner pharmazeutisch verträglichen Salze in Form eines transdermalen therapeutischen Systems, das Desoxypeganin und / oder dessen pharmazeutisch verträgliches Salz über einen Zeitraum von mindestens 24 Stunden kontrolliert abgibt und gewährleistet, dass das Desoxypeganin sich während der Lagerung des vorgefertigten transdermalen therapeutischen Systems nicht merklich zersetzt und sicherstellt, dass das Desoxypeganin im geforderten Ausmass in vivo durch menschliche Haut penetriert.

Diese Aufgabe wird mit der Erfindung in überraschender Weise gelöst durch ein transdermales therapeutisches System, welches eine für Desoxypeganin oder eines seiner pharmazeutisch verträglichen Salze undurchlässige Rückschicht und eine haftklebende Reservoirschicht enthält. Diese Reservoirschicht enthält 10-80 Gew.-% Polymermaterial, 0,1-30 Gew.-% Desoxypeganin und / oder eines seiner pharmazeutisch verträglichen Salze und gegebenenfalls einen Weichmacher in Anteilen von 0,1-30 Gew.-%.

Merkmale weiterer vorteilhafter Ausgestaltungsformen eines erfindungsgemäßen transdermalen therapeutischen Systems sind im weiteren beschrieben.

Diese Lösung ist umso erstaunlicher, als Desoxypeganin strukturell den Tricyclen zuzuordnen ist. Tricyclen sind eine Substanzklasse, die als die menschliche Haut nur in unzureichendem Masse zu durchdringen vermögend gilt.

Ohne den Rahmen der Erfindung einzuschränken, sollen unter pharmazeutisch verträglichen Salzen des Desoxypeganins vorzugsweise dessen Hydrobromid und Hydrochlorid verstanden werden.

Die wirkstoffundurchlässige Rücksicht kann aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden.

Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einer mit Aluminium bedampften Folie.

Die Reservoirschicht besteht aus einer Polymermatrix und dem Wirkstoff, wobei die Polymermatrix den Zusammenhalt des Systems gewährleistet. Sie besteht aus einem Grundpolymer und gegebenenfalls den üblichen Zusätzen. Die Auswahl des Grundpolymers richtet sich nach den chemischen und physikalischen Eigenschaften des Desoxypeganins. Beispielhafte Grundpolymere sind Kautschuk, kautschukähnliche, synthetische Homo-, Co- oder Blockpolymere, Polyacrylsäureester und deren Copolymere, Polyurethane und Silikone. Grundsätzlich kommen alle Polymere in Frage, die bei der Herstellung von Haftklebern eingesetzt werden können und physiologisch unbedenklich sind. Besonders bevorzugt sind solche, die aus Blockcopolymeren auf Basis von Styrol und 1,3-Dienen, Polyisobutylenen, Silikonen, Polymeren auf Acrylat- und / oder Methacrylat-Basis bestehen.

Von den Blockcopolymeren auf Basis von Styrol und 1,3-Dienen werden ganz besonders lineare Styrol-lsopren- oder Styrol-Butadien-Blockcopolymere eingesetzt.

Als Polymere auf Acrylat-Basis werden selbstvernetzende Acrylatcopolymere aus 2-Ethylhexylacrylat, Vinylacetat und Acrylsäure mit bzw. nicht selbstvernetzende Acrylatcopolymere ohne Titanchelatester bevorzugt.

Als Polymere, die dem Grundpolymer zugesetzt werden, kommen Polymethacrylate und Polyvinyle in Frage. Als Methacrylate werden Copolymere auf Basis von Dimethylaminoethylmethacrylaten und neutralen Methacrylsäureestern bevorzugt. Als Polyvinyle werden vorzugsweise Polyvinylpyrrolidone und Polyvinylalkohole eingesetzt.

Als besonders vorteilhaft haben sich Cellulosederivate als Bestandteile des Polymermaterials erwiesen.

Die Wahl des Weichmachers richtet sich nach dem Polymer. Besonders geeignet sind höhere Alkohole wie Dodecanol, Undecanol, Octanol, Oleylalkohol und 2-Octyldodecanol, Ester von Carbonsäuren (z.B. Isopropylmyristat), wobei die Alkoholkomponente auch ein polyethoxylierter Alkohol sein kann, Diester von Dicarbonsäuren, z. B. Di-n-butyladipat sowie Triglyceride, insbesondere mittelkettige Triglyceride der Capryl/Caprinsäuren des Kokosöls. Weitere Beispiele für einen geeigneten Weichmacher sind mehrwertige Alkohole, z. B. Glycerin und Propandiol-(1, 2) u. a., die auch durch Polyethylenglykole verethert sein können.

Als Penetrationsförderer, die in einer weiteren Ausführungsform des erfindungsgemäßen transdermalen therapeutischen Systems eingesetzt werden können, kommen alle Carbonsäuren in Frage, die physiologisch unbedenklich sind. Besonders geeignet sind Octansäure, Lävulinsäure, Laurinsäure, Undecensäure, Ölsäure sowie Stearinsäure und ihre Isomeren.

Die Art der in weiteren Ausführungsformen verwendeten üblichen Zusätze hängt vom eingesetzten Polymer ab: Nach ihrer Funktion lassen sie sich einteilen in beispielsweise Klebrigmacher, Stabilisatoren, Trägerstoffe und Füllstoffe. Die hierfür in Frage kommenden physiologisch unbedenklichen Substanzen sind dem Fachmann bekannt.

Die Reservoirschicht besitzt eine solche Eigenklebrigkeit, dass ein während der Anwendungsdauer des erfindungsgemäßen TTS bestehender inniger Kontakt zur Haut des Patienten sichergestellt ist.

Eine ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, dass sie ablösbar gemacht werden, wie z. B. durch eine Siliconbehandlung. Andere ablösbare Schutzschichten sind z. B. Polytetrafluorethylen, behandeltes Papier, Cellophan, Polyvinylchlorid u. ä. Wird das erfindungsgemässe Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

in weiteren erfindungsgemäßen Ausführungsformen kann die Reservoirschicht auch durch eine die Freisetzung des Desoxypeganin und / oder eines seiner pharmazeutisch verträglichen Salze kontrollierenden Membran, z. B. einer mikroporösen oder einer semipermeablen Membran, abgedeckt sein. Sofern diese Membran nicht haftklebend ist, kann eine weitere Haftkleberschicht den Hautkontakt sicherstellen.

Das erfindungsgemässe transdermale therapeutische System wird hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das oder die Lösemittel entfernt wird / werden. Anschliessend wird die Klebeschicht mit einer entsprechenden Schutzschicht versehen, ggf. nachdem eine die Freisetzungsgeschwindigkeit kontrollierende Membran darüberkaschiert worden ist.

Auch der umgekehrte Weg, dass die Kleberlösung auf die Schutzschicht aufgestrichen wird, ist grundsätzlich möglich. Man entfernt auch in diesem Fall die Lösungsmittel und deckt dann mit der Rückschicht ab.

Die Erfindung wird durch die folgenden Beispiele erläutert:

### Beispiel 1

1,0 g Laurinsäure und 0,5 g Isopropylmyristat werden unter Rühren gemischt. Anschliessend werden 1,0 g Desoxypeganin eingetragen; man rührt bis zum vollständigen Auflösen des Feststoffs (ca. 30 min, visuelle Kontrolle). Danach werden unter Rühren 1,625 g Ethylcellulose, gelöst in 6,25 g Ethylacetat zugegeben; es wird homogenisiert. Danach werden unter Rühren noch zusätzlich 4,5 g Abitol und 1,25 g Hercures C, gelöst in 1,25 g Benzin zugefügt. Es wird 3 Stunden lang bei Raumtemperatur gerührt. Der Verdunstungsverlust wird ausgeglichen.

Es resultieren 17,375 g einer 56,82%igen (G/G) wirkstoffhaltige Kleberlösung, die mit einem 350 µm Rakel auf eine aluminisierte und silikonisierte Polyethylenfolie gestrichen wird. Nachdem die Lösungsmittel durch 30minütiges Trocknen bei 60°C entfernt wurden, deckt man den Kleberfilm mit einer Polyesterfolie 15 µm ab. Mit geeigneten Schneidewerkzeugen stanzt man eine Fläche von 16 cm² aus und entfernt die Ränder durch Abgittern (Entfernen überflüssiger Matrixteile). Die Freisetzung dieses und der anderen Rezepturbeispiele sind in den Abbildungen wiedergegeben; dort ist sowohl die kontrollierte Freisetzung in eine physiologische Kochsalzlösung als auch die durch Humanhaut aufgeführt.

### Beispiel 2

Das TTS dieses Beispiels wird nach dem unter Beispiel 1 angegebenen Schema angefertigt, jedoch ohne Verwendung von Laurinsäure. In der folgenden Tabelle sind die Rezepturbestandteile nach Trocknen aufgeführt.

| Desoxypeganin-Rezepturen | | |
|---|---|---|
| Eingesetztes Material | Versuch 1, Gehalt [%] | Versuch 2, Gehalt [%] |
| Ethylacetat | - | - |
| Benzin | - | - |
| Isopropylmyristat | 5,06 | 5,06 |
| Hercures C | 12,66 | 13,92 |
| Abitol | 45,57 | 50,63 |
| Desoxypeganin | 10,12 | 10,12 |
| Ethylcellulose | 16,45 | 20,25 |
| Laurinsäure | 10,12 | - |

Die in vitro-Freisetzung wurde in einem Schüttelwasserbad bei 37°C bestimmt.

Das Akzeptormedium waren 100 ml physiologische Kochsalzlösung, die nach 2, 4 und 8 Stunden komplett ausgewechselt wurden. Die Konzentration wurde nach 2, 4 und 8 und 24 Stunden per HPLC bestimmt. Die Penetration an der Humanhaut wurde an Franz'schen Diffusionszellen gemessen.

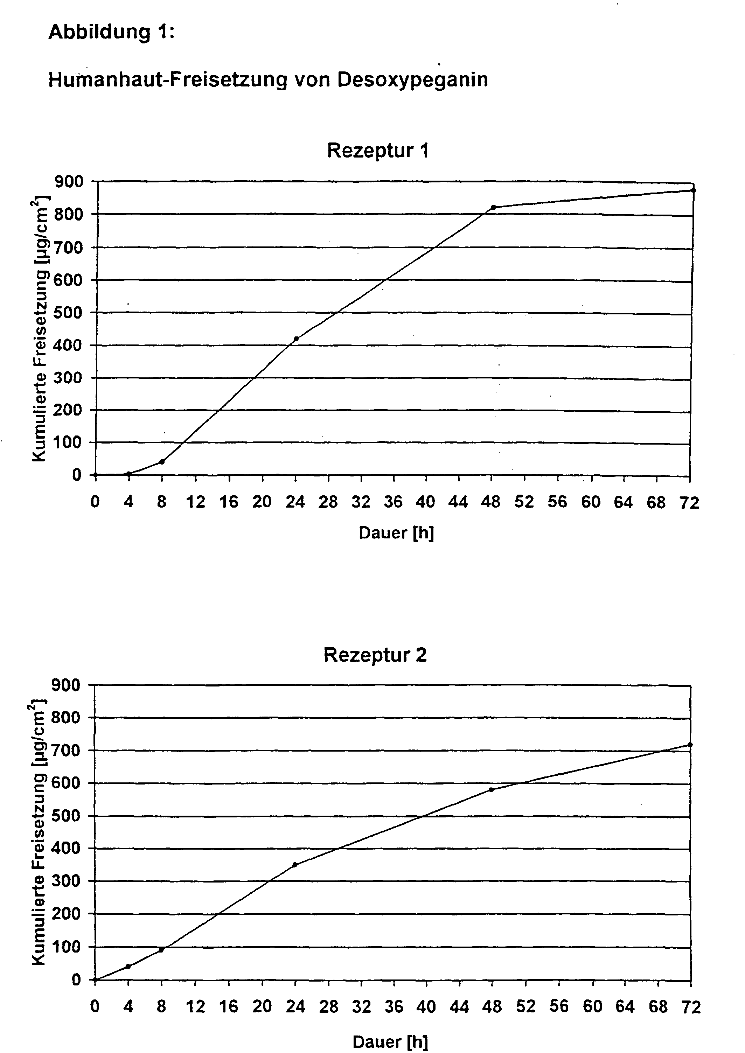

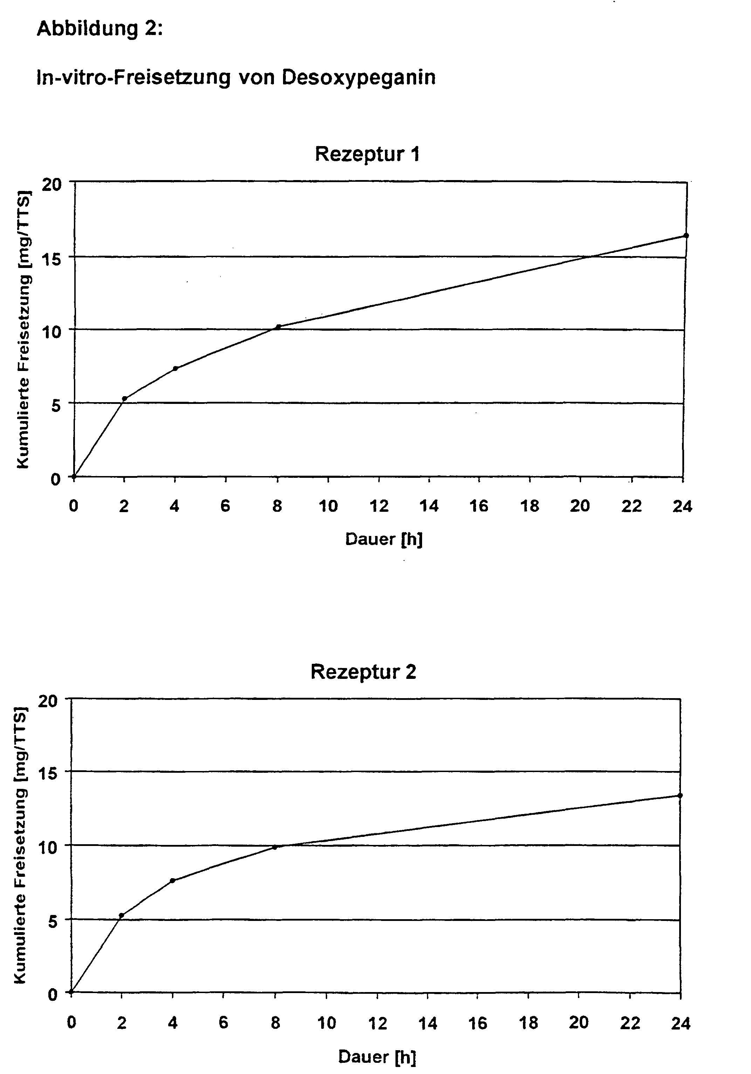

## Patentansprüche

1. Transdermales therapeutisches System (TTS) zur Verabreichung von Desoxypeganin an die Haut mit einer wirkstoffundurchlässigen Rückschicht und einer haftklebenden Reservoirschicht, **dadurch gekennzeichnet, dass** die Reservoirschicht 10-80 Gew.-% Polymermaterial und 0,1-30 Gew.-% Desoxypeganin und / oder eines seiner pharmazeutisch verträglichen Salze enthält.

2. TTS nach Anspruch 1, **dadurch gekennzeichnet, dass** zusätzlich eine die Freisetzung des Wirkstoffs kontrollierende, semipermeable oder mikroporöse Membran enthalten ist.

3. TTS nach Anspruch 1 oder 2, **dadurch gekennzeichnet, dass** die haftklebende Reservoirschicht 0,1-30 Gew.-% eines Penetrationsförderers enthält.

4. TTS nach Anspruch 3, **dadurch gekennzeichnet, dass** der Penetrationsförderer eine Carbonsäure ist.

5. TTS nach Anspruch 1 oder 2, **dadurch gekennzeichnet, dass** die haftklebende Reservoirschicht 0,1-30 Gew.-% eines Weichmachers enthält.

6. TTS nach Anspruch 1, **dadurch gekennzeichnet, dass** das Polymermaterial aus der Gruppe der Polyacrylate und der Pofymerisationsprodukte von Acrylsäure und / oder Methacrylsäure und / oder ihren Estern ausgewählt ist.

7. TTS nach Anspruch 6, **dadurch gekennzeichnet, dass** das Polymermaterial unter Verwendung von Estern der Acrylsäure, die als alkoholische Komponente geradkettige oder verzweigte Alkohole mit 4-10 Kohlenstoffatomen enthalten, hergestellt wurde.

8. TTS nach Anspruch 6, **dadurch gekennzeichnet, dass** das Polymermaterial unter Verwendung von Estern der Acrylsäure, die als alkoholische Komponente geradkettige oder verzweigte Alkohole mit 2-4 Kohlenstoffatomen enthalten, hergestellt wurde.

9. TTS nach Anspruch 6, **dadurch gekennzeichnet, dass** das Polymermaterial unter Verwendung von Estern der Methacrylsäure, die als alkoholische Komponente Aminoalkohole enthalten, hergestellt wurde.

10. TTS nach Anspruch 6, **dadurch gekennzeichnet, dass** das Polymermaterial selbstvernetzende oder nicht-selbstvernetzende Acrylatcopolymere enthält.

11. TTS nach Anspruch 1 oder 2, **dadurch gekennzeichnet, dass** die den Wirkstoff freisetzende Fläche mit einer wiederablösbaren Schutzschicht versehen ist.

12. TTS nach Anspruch 1 oder 2, **dadurch gekennzeichnet, dass** das Polymermaterial Cellulose und seine Derivate enthält.

13. Verwendung von Desoxypeganin zur Herstellung eines TTS mit einer wirkstoffundurchlässigen Rückschicht und einer haftklebenden Reservoirschicht, **dadurch gekennzeichnet, dass** die Reservoirschicht 10-80 Gew.-% Polymermaterial und 0,1-30 Gew.-% Desoxypeganin und / oder eines seiner pharmazeutisch verträglichen Salze enthält.

## Claims

1. Transdermal therapeutic system (TTS) for the administration of deoxypeganine to the skin, having an active-compound-impermeable back layer and a contact-adhesive reservoir layer, **characterized in that** the reservoir layer comprises 10-80% by weight of polymer material and 0.1-30% by weight of deoxypeganine and/or one of its pharmaceutically tolerable salts.

2. TTS according to Claim 1, **characterized in that** a semipermeable or microporous membrane controlling the release of the active compound is additionally present.

3. TTS according to Claim 1 or 2, **characterized in that** the contact-adhesive reservoir layer comprises 0.1-30% by weight of a penetration promoter.

4. TTS according to Claim 3, **characterized in that** the penetration promoter is a carboxylic acid.

5. TTS according to Claim 1 or 2, **characterized in that** the contact-adhesive reservoir layer comprises 0.1-30% by weight of a plasticizer.

6. TTS according to Claim 1, **characterized in that** the polymer material is selected from the group of polyacrylates and the polymerization products of acrylic acid and/or methacrylic acid and/or their esters.

7. TTS according to Claim 6, **characterized in that** the polymer material was produced using esters of acrylic acid which, as alcoholic components, comprise straight-chain or branched alcohols having 4-10 carbon atoms.

8. TTS according to Claim 6, **characterized in that** the polymer material was produced using esters of acrylic acid which, as alcoholic components, comprise straight-chain or branched alcohols having 2-4 carbon atoms.

9. TTS according to Claim 6, **characterized in that** the polymer material was produced using esters of methacrylic acid which, as alcoholic components, comprise aminoalcohols.

10. TTS according to Claim 6, **characterized in that** the polymer material comprises self-crosslinking or non self-crosslinking acrylate copolymers.

11. TTS according to Claim 1 or 2, **characterized in that** the area releasing the active compound is provided with a protective layer which can be removed again.

12. TTS according to Claim 1 or 2, **characterized in that** the polymer material comprises cellulose and its derivatives.

13. Use of deoxypeganine for the production of a TTS having an active-compound-impermeable back layer and a contact-adhesive reservoir layer, **characterized in that** the reservoir layer comprises 10-80% by weight of polymer material and 0.1-30% by weight of deoxypeganine and/or one of its pharmaceutically tolerable salts.

## Revendications

1. Système thérapeutique transdermique (STT) pour l'administration de désoxypéganine à la peau avec une couche, au dos, imperméable à la substance active et une couche réservoir autoadhésive, **caractérisé en ce que** la couche réservoir contient de 10 à 80% en poids de matière polymère et de 0,1 à 30% en poids de désoxypéganine et/ou d'un de ses sels pharmaceutiquement acceptable.

2. STT selon la revendication 1, **caractérisé en ce qu'**il contient en plus une membrane semi-perméable ou microporeuse, contrôlant la libération de la substance active.

3. STT selon la revendication 1 ou 2, **caractérisé en ce que** la couche réservoir autoadhésive contient de 0,1 à 30% en poids d'un promoteur de pénétration.

4. STT selon la revendication 3, **caractérisé en ce que** le promoteur de pénétration est un acide carboxylique.

5. STT selon la revendication 1 ou 2, **caractérisé en ce que** la couche réservoir autoadhésive contient de 0,1 à 30% en poids d'un plastifiant.

6. STT selon la revendication 1, **caractérisé en ce que** la matière polymère est choisie dans le groupe des polyacrylates et des produits de polymérisation des acides acryliques et/ou des acides méthacryliques et/ou de leurs esters.

7. STT selon la revendication 6, **caractérisé en ce que** la matière polymère a été préparée en utilisant des esters d'acides acryliques qui contiennent des alcools linéaires ou ramifiés de 4 à 10 atomes de carbone en tant que composante alcoolique.

8. STT selon la revendication 6, **caractérisé en ce que** la matière polymère a été préparée en utilisant des esters d'acides acryliques qui contiennent des alcools linéaires ou ramifiés de 2 à 4 atomes de carbone en tant que composante alcoolique.

9. STT selon la revendication 6, **caractérisé en ce que** la matière polymère a été préparée en utilisant des esters d'acides méthacryliques qui contiennent des alcools aminés en tant que composante alcoolique.

10. STT selon la revendication 6, **caractérisé en ce que** la matière polymère contient des copolymères acryliques autoréticulants ou non-autoréticulants.

11. STT selon la revendication 1 ou 2, **caractérisé en ce que** la surface de libération de la substance active est munie d'une couche de protection amovible.

12. STT selon la revendication 1 ou 2, **caractérisé en ce que** la matière polymère contient de la cellulose et ses dérivés.

13. Utilisation de désoxypéganine pour la fabrication d'un STT avec une couche, au dos, imperméable à la substance active et une couche réservoir autoadhésive, **caractérisée en ce que** la couche réservoir contient de 10 à 80% en poids de matière polymère et de 0,1 à 30% en poids de désoxypéganine et/ou d'un de ses sels pharmaceutiquement acceptable.
